# EUROPEAN PATENT SPECIFICATION

(11) **EP 4 295 770 B1**
(45) Date of publication and mention of the grant of the patent: **04.03.2026**
(21) Application number: 22755882.2
(22) Date of filing: 27.01.2022
(51) Int. Cl.: A61B 5/25, A61B 5/369

(54) **BIOELECTRODE**
BIOELEKTRODE
BIOÉLECTRODE

(30) Priority: 18.02.2021 JP 2021024067
(43) Date of publication of application: 27.12.2023
(73) Proprietor: NOK Corporation, Tokyo 105-8585 (JP)
(72) Inventor: KUBO, Masayuki, Tsujido-shinmachi, Fujisawa-shi, Kanagawa 251-0042 (JP); FUTASHIMA, Ryo, Tsujido-shinmachi, Fujisawa-shi, Kanagawa 251-0042 (JP); HAYASHI, Takahiro, Tsujido-shinmachi, Fujisawa-shi, Kanagawa 251-0042 (JP)
(74) Representative: TBK
(86) International application number: PCT/JP2022/003137
(87) International publication number: WO 2022/176559

(56) References cited:
- EP-A1- 4 449 990
- WO-A1-2016/136629
- WO-A1-2017/065196
- WO-A1-2018/230445
- JP-A- 2003 260 034
- JP-A- 2014 512 211
- US-A1- 2020 029 881
- ANONYMOUS: "I was surprised by NOK's oil seal "Le-μ's" (Lemuse). And it's not just the oi flexible substrates to rubber electrodes for electroencephalograms", XP055961177, Retrieved from the Internet <URL:https://car.motor-fan.jp/article/photo/10012381/2013594201911131909450000001> [retrieved on 20220914]

## Description

### Technical Field

The present invention relates to a biological electrode, and more particularly, to a biological electrode capable of realizing good contacting with a scalp of a subject for detection.

### Description of the Related Art

In order to diagnose a health condition of a subject for detection in a medical facility or the like, a biological electrode is disposed on a body of the subject to detect various electric signals. For example, electroencephalogram is measured by disposing an electrode on a scalp of the subject.

Conventionally, as an electrode for electroencephalography, for example, a biological electrode including an electrode portion made of a conductive silicone rubber or the like has been proposed (for example, see Patent Document 1). Examples of the electrode portion of the biological electrode include those in which a plurality of protrusions with sharp tips are provided on the side that comes into contact with a subject, and the plurality of protrusions are formed in a brush-like shape. The biological electrode for electroencephalography further includes, for example, a pedestal for disposing an electrode portion, and the pedestal is provided with a terminal for assembling with a counterpart component, an insulating rubber for supporting the terminal, and the like.

Conventionally, in a biological electrode for electroencephalography, the number of protrusions at the tips of the electrode portion is set to be larger in order to increase the area in contact with a scalp of a subject. Hereinafter, the number of protrusions provided in the electrode portion may be referred to as the "number of protrusions" of the electrode portion.

### Citation List

### Patent Document

[Patent Document 1] JP-A-2017-74369
WO 2017/065196 A1 discloses a biological electrode according to the preamble of claim 1. Further biological electrodes are known from US 2020/029881 A1 and WO 2016/136629 A1.

### SUMMARY OF THE INVENTION

### Problem to be Solved by the Invention

However, when the number of protrusions of the electrode portion in the biological electrode for electroencephalography is increased, the protrusions are densely arranged with respect to one electrode portion, and therefore, when the electroencephalogram of a subject having a large amount of hair is measured, a space for housing the hair is reduced between the scalp and the electrode portion. For this reason, such a biological electrode is problematic in that the electrode portion easily floats from the scalp, and conversely, it is difficult to contact with the scalp.

In addition, when detecting an electroencephalogram of a subject having a large amount of hair, a strong pressing force must be applied to a biological electrode in order to prevent the biological electrode from floating due to the hair and to bring the electrode portions into close contact with a scalp of the subject. However, if the use of biological electrode with a strong pressing force is continued for a long time as described above, the scalp of the subject may be painful.

In view of the above problems, the present invention provides a biological electrode capable of realizing good contacting with the scalp of a subject. In particular, there is provided a biological electrode suitable for electroencephalography and capable of realizing good contacting with the scalp even in a subject having a large amount of hair.

### Means for Solving the Problems

In order to solve the above problem, the present invention provides the biological electrode according to claim 1. Further advantageous developments of the invention are subject-matter of the dependent claims.

### Effect of the Invention

The biological electrode of the present invention is effective in realizing good contacting with a scalp of a subject. In addition, the biological electrode of the present invention does not require excessive pressing of the electrodes. Therefore, it is possible to reduce pain in the scalp at the time of measuring. In particular, even in a subject having a large amount of hair, a subject having long hair, a subject having a large volume of hair (in other words, having thick hair), or the like, stable contacts with the scalp can be realized.

### BRIEF DESCRIPTION OF THE DRAWINGS

FIG. 1 is a front view schematically showing a biological electrode according to an embodiment of the present invention.
FIG. 2 is a bottom view of the biological electrode shown in FIG. 1.
FIG. 3 is a cross-sectional view taken along the line A-A in FIG. 2.
FIG. 4 is an explanatory view showing an example of using a biological electrode for electroencephalography.

### Mode for Carrying out the Invention

Hereinafter, embodiments of the present invention will be described with reference to the drawings. It should be understood that the present invention is not limited to the following embodiments, and changes, improvements, and the like of the design can be appropriately made based on ordinary knowledge of a person skilled in the art without departing from the spirit of the present invention.

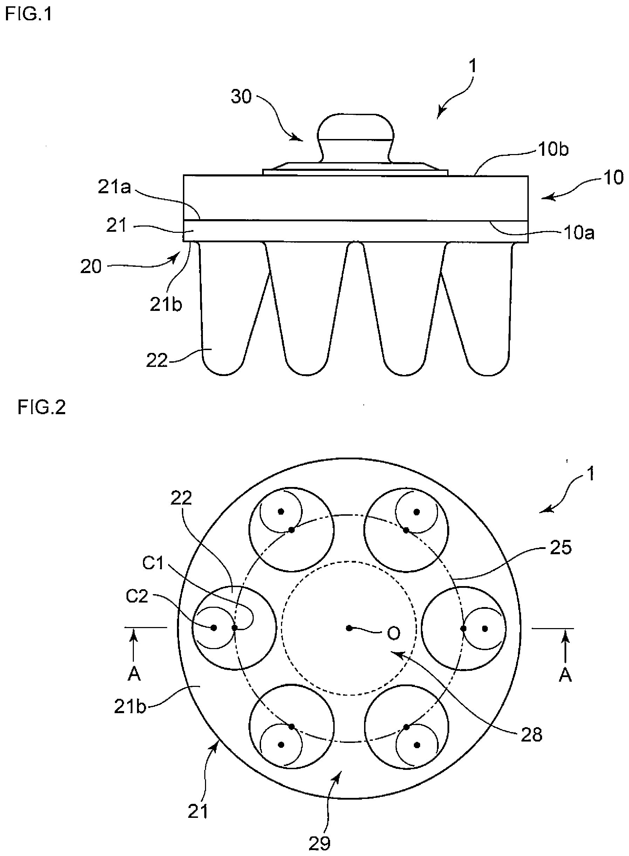

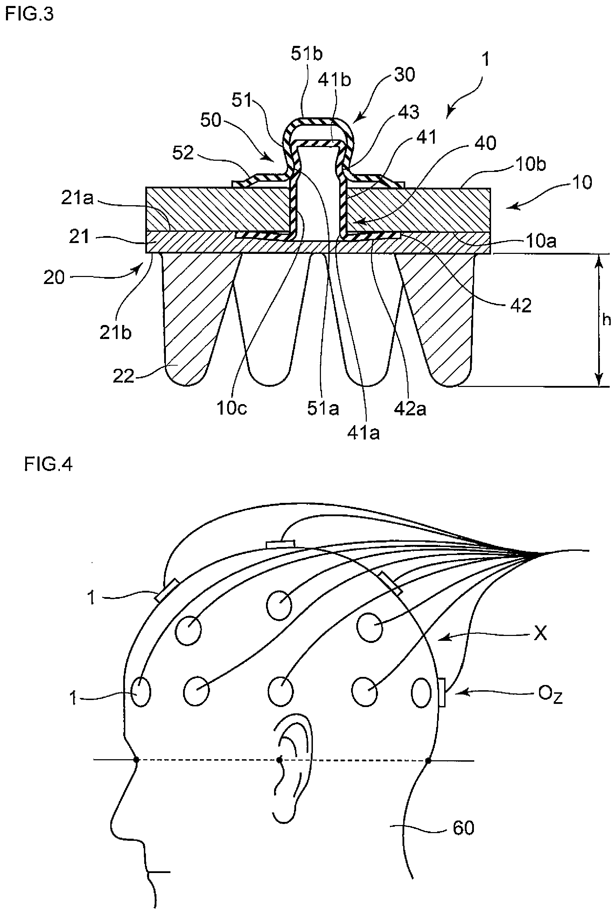

FIG. 1 is a front view schematically showing a biological electrode according to an embodiment of the present invention. FIG. 2 is a bottom view of the biological electrode shown in FIG. 1. FIG. 3 is a cross-sectional view taken along the line A-A in FIG. 2. FIG. 4 is an explanatory view showing an example of using a biological electrode for electroencephalography.

As shown in FIGS. 1 to 3, the biological electrode 1 includes a electrode member 20 having an electrode body 21 having a plate shape and a plurality of electrode protrusions 22 provided so as to protrude from an electrode protrusion forming surface 21b of the electrode body 21. The electrode member 20 can detect (extract) a biological signal of a subject via a connector, for example, by contacting a distal end side of the plurality of electrode protrusions 22 with a body (skin) of a subject. The biological electrode 1 is used, for example, as a biological electrode for electroencephalography. In this case, the biological electrode 1 is attached to the head of the subject such that the distal end side of the plurality of electrode protrusions 22 in the electrode member 20 comes into contact with the scalp of the subject. For example, as shown in FIG. 4, various electric signals such as the electroencephalogram of a subject 60 can be detected by arranging a plurality of biological electrodes 1 at desired positions on the head of the subject 60.

The biological electrode 1 can be suitably used for sensing electrical signals from a body of a subject, transmitting electrical stimulations to a subject, or both the sensing and transmitting described above, by contacting a plurality of electrode protrusions 22 of the electrode member 20 with a body of a subject. Specifically, for example, it can be used as a biological electrode 1 for a medical measuring instrument, a wearable measuring instrument, a health monitoring instrument, or the like. In particular, the biological electrode 1 can be suitably used when measuring electroencephalograms as electric signals.

The biological electrode 1 of the present embodiment further includes a support member 10 for supporting the electrode member 20. The support member 10 is made of an electrically insulating material. For example, the support member 10 may be formed of a silicone rubber or the like. In the biological electrode 1 of the present embodiment, the support member 10 is formed in a disk shape. The support member 10 has a support surface 10a for supporting the electrode member 20 and a rear surface 10b opposed to the support surface 10a. In addition, a through hole 10c that penetrates the support member 10 in the thickness direction (that is, penetrates from the support surface 10a to the rear surface 10b) is formed in the center part of the support member 10 (see, for example, FIG. 3).

Note that the support member 10 only needs to have a configuration corresponding to the support surface 10a, the rear surface 10b, and the through hole 10c, and is not necessarily formed in a disk shape.

In the biological electrode 1, the electrode member 20 is made of, for example, a conductive rubber, and has, as described above, an electrode body 21 supported by the support member 10, and a plurality of electrode protrusions 22 protruding from the electrode body 21 on the side opposite to the support member 10. Examples of the conductive rubber include so-called conductive silicone rubbers containing silicone rubber and metal particles. Examples of the silicone rubber include a room temperature curable type liquid silicone rubber. Further, examples of the metal particles include silver particles. The room temperature curable type liquid silicone rubber is a silicone rubber that is in a liquid state or a paste state before curing, and that undergoes a curing reaction at 20°C to 100°C to become a rubber elastic body. The silver particles may include aggregated particles (aggregates) in which a plurality of silver particles (primary particles) are gathered and aggregated, and silver particles in a flake state.

The conductive rubber forming the electrode member 20 may include other conductive metal particles, carbon-based material particles (such as a carbon black and a carbon nanotube), or the like, instead of the silver particles, and may appropriately include a reinforcing material, a filler, various additives, and the like.

The electrode body 21 of the electrode member 20 preferably has a shape similar to that of the support member 10. That is, in the biological electrode 1 of the present embodiment, the electrode body 21 is formed in a disk shape. The electrode body 21 has a supported surface 21a supported by the support surface 10a of the support member 10 and an electrode protrusion forming surface 21b opposed to the supported surface 21a.

The plurality of electrode protrusions 22 are protruded on the electrode protrusion forming surface 21b of the electrode body 21. The plurality of electrode protrusions 22 are arranged on the outer peripheral part 29 of the electrode protrusion forming surface 21b so as to avoid the central part 28 of the electrode protrusion forming surface 21b of the electrode body 21.

In the biological electrode 1 of the present embodiment, the protrusion height h of each electrode protrusion 22 arranged on the outer peripheral part 29 of the electrode protrusion forming surface 21b as described above has a particularly main configuration. That is, in the biological electrode 1 of the present embodiment, the protrusion height h of each electrode protrusion 22 from a proximal end thereof to a distal end thereof in the direction perpendicular to the electrode protrusion forming surface 21b is 6 to 15mm. Hereinafter, the protrusion height h of each electrode protrusion 22 from a proximal end thereof to a distal end thereof in a direction perpendicular to the electrode protrusion forming surface 21b may be simply referred to as a "protrusion height h of the electrode protrusion 22".

In the biological electrode 1, since the electrode protrusions 22 having a protrusion height h of 6 to 15mm are arranged only on the outer peripheral part 29 of the electrode protrusion forming surface 21b, the central part 28 of the electrode protrusion forming surface 21b surrounded by the electrode protrusions 22 is a space for housing the hair of the subject. The space in the central part 28 has a space height corresponding to the protrusion height h of the electrode protrusion 22, and the hair of the subject can be satisfactorily housed in the space. Therefore, the biological electrode 1 can realize good contact with the scalp of a subject, and the excessive pressing of the biological electrode 1 against the subject is not required, and the pain of the scalp at the time of measuring can be reduced. In particular, even in a subject having a large amount of hair, a subject having long hair (for example, a woman growing her hair), a subject having a large volume of hair (in other words, having thick hair), or the like, stable contacts with the scalp can be realized.

If the protrusion height h of the electrode protrusion 22 is less than 6mm, the space height of the central part 28 of the electrode protrusion forming surface 21b is too low, and the hair of the subject may not be sufficiently housed in the space of the central part 28. For example, generally, the average hair thickness is 0.085mm, and the number of hairs growing per unit area of the scalp of the subject is on the order of 208 hairs/cm². Hereinafter, the number of hairs growing per unit area may be referred to as "density of hair (hairs/cm²)".

As shown in FIG. 4, when a plurality of biological electrode 1 are arranged on the head of the subject 60 to detect electric signals such as an electroencephalogram, it can be said that the occiput of the subject 60 indicated by the measurement point Oz is a part where the biological electrode 1 is more likely to float due to the influence of hair of the upper part or the like. Then, the point x in FIG. 4 is in a range that is assumed to be affected by overlapping hair at the time of measurement at the measurement point Oz, and the length from the measurement point Oz to the point x is 5mm. For this reason, when the length of the range affected by overlapping hair is defined as 5mm, according to the average hair thickness (mm) and the density of hair (hairs/cm²) with respect to the general subject described above, the number of hairs per unit length is 14 hairs/cm, the number of hairs in the linear range up to the range (5mm) is 72, and when all the hair in the linear range up to the range (5mm) is overlapped, the hair bulk (in other words, the height of the overlapped hair) is 6mm. Therefore, by setting the protrusion height h of the electrode protrusion 22 to be equal to or greater than 6mm, it is possible to satisfactorily house the hair of the subject in the space of the central part 28 surrounded by the electrode protrusions 22.

In a subject having thick hair and high hair density, the average hair thickness is about 0.150mm, and the hair density is about 300 hairs/cm². Then, according to the average hair thickness (mm) and the hair density (hairs/cm²) for such a subject, the number of hairs per unit length is 17 hairs/cm, the number of hairs in the linear line of the above range (5mm) is 87, and when all the hair in the linear line up to the above range (5mm) is overlapped, the hair bulk is 13mm. Therefore, if the protrusion height h of the electrode protrusion 22 is secured to 15mm, even in a subject having thick hair and high hair density, the hair can be satisfactorily housed in the space of the central part 28 surrounded by the electrode protrusions 22. Then, if the protrusion height h of the electrode protrusion 22 exceeds 15mm, the protrusion height h of the electrode protrusion 22 becomes excessive, and the demoldability from the mold when the electrode protrusion 22 is molded and formed may deteriorate. In addition, from the viewpoint of reducing the size of the biological electrode 1, it is not preferable that the protrusion height h of the electrode protrusion 22 becomes excessive.

The arrangement of the plurality of electrode protrusions 22 in the outer peripheral part 29 of the electrode protrusion forming surface 21b is preferably circular with respect to the outer peripheral part 29 of the electrode protrusion forming surface 21b from the viewpoint of balancing. For example, each of the plurality of electrode protrusions 22 is preferably arranged so as to be located on the circumference of the virtual circle 25 on the outer peripheral part 29 of the electrode protrusion forming surface 21b. The virtual circle may be one virtual circle or a plurality of virtual concentric circles. However, the arrangement of the electrode protrusions 22 is not limited to the above-described arrangement, and for example, a shape drawn by connecting the points where a plurality of electrode protrusions 22 are arranged may be a triangle, a quadrangle, or the like.

The plurality of electrode protrusions 22 are preferably arranged at equal intervals on the outer peripheral part 29 of the electrode protrusion forming surface 21b. It should be noted that the distance between two adjacent electrode protrusions 22 is not necessary to be strictly equal, and may be substantially equal. Further, the arrangement of the electrode protrusion 22 is not limited to the above-described aspect, and may be, for example, such that, in the outer peripheral part 29 of the electrode protrusion forming surface 21b, all of the distances between two adjacent electrode protrusions 22 are not the same, and for example, they may have a constant arrangement pattern having two or more types of distances. Further, for example, a plurality of electrode protrusions 22 may be randomly arranged on the outer peripheral part 29 of the electrode protrusion forming surface 21b.

The number of the electrode protrusions 22 arranged on the outer peripheral part 29 of the electrode protrusion forming surface 21b is not particularly limited, and can be appropriately determined in accordance with the size of the electrode protrusion forming surface 21b and the like.

Further, the shapes of the electrode protrusion forming surface 21b are not particularly limited. The protrusions may be formed so as to protrude from the electrode protrusion forming surface 21b of the electrode body 21 so that the protrusion height h is 6 to 15mm. Here, an example of the shape of the electrode protrusion forming surface 21b will be described with reference to an example of the electrode protrusion forming surface 21b in the biological electrode 1 shown in FIGS. 1 to 3.

As shown in the biological electrode 1 of FIGS. 1 to 3, for example, each of the plurality of electrode protrusion 22 is preferably formed so that the cross-sectional area gradually decreases from a proximal end (root) toward a distal end, in other words, as the distance from the electrode protrusion forming surface 21b increases. For example, each of the plurality of electrode protrusions 22 has a circular cross section and the cross section gradually decreases in diameter from the proximal end (root) toward the distal end. It is preferable that the center C1 of the cross section of each proximal end of the plurality of electrode protrusions 22 is arranged on the circumference of the virtual circle 25 on the outer peripheral part 29 as described above. In such a configuration, the center C1 of the cross section of each proximal end of the plurality of electrode protrusions 22 is not necessary to be strictly located on the circumference of the virtual circle 25, and may be located substantially on the circumference thereof.

Each distal end of the plurality of electrode protrusions 22 is preferably formed in a hemispherical shape. In addition, each of the plurality of electrode protrusions 22 is preferably formed such that, when viewed from the arrangement center O of the plurality of electrode protrusions 22, the center of the distal end (also referred to as the center of the cross section of the distal end) C2 is located radially outward of the center C1 of the cross section of the proximal end. For example, such an electrode protrusion 22 has an obliquely conical shape with a rounded apex of the distal end. With this configuration, it is possible to secure a wider space of the central part 28 of the electrode protrusion forming surface 21b surrounded by the electrode protrusions 22.

In the biological electrode 1, the connector 30 is formed as a snap button type connector. More specifically, the connector 30 is formed as a male side connector in a snap button type connector. For example, as shown in FIG. 3, the connector 30 includes a first conductive member 40 and a second conductive member 50 fitted to each other.

The first conductive member 40 and the second conductive member 50 are formed of stainless steel, for example. In the first conductive member 40, one end side is embedded in the electrode body 21 of the electrode member 20 and extends through the support member 10, and the other end side protrudes from the rear surface 10b of the support member 10. The second conductive member 50 is disposed on the rear surface 10b of the support member 10, in a state of being fitted to the other end side of the first conductive member 40. Then, the electrode member 20 of the biological electrode 1 is electrically connected to the outside by mounting (fitting) the female side connector (not shown) of the snap button type connector to the second conductive member 50. That is, the connector 30 is configured such that a part thereof is embedded in the electrode body 21 of the electrode member 20 and extends through the support member 10 to have a connecting part with the outside which is positioned on the rear surface 10b of the support member 10.

For example, the electrode member 20 of the biological electrode 1 is electrically connected to the measuring device by mounting (fitting) a female side connector attached to the distal end of a lead wire of the measuring device to the second conductive member 50. The measuring device is a device for inputting a biological signal detected by the plurality of electrode protrusions 22 of the electrode member 20 of the biological electrode 1, and processing, displaying, and analyzing the input biological signal. and is not particularly limited, but may be, for example, an electroencephalogram measuring device, a wearable information device, and a health monitoring device.

The first conductive member 40 and the second conductive member 50 will be specifically described with reference to FIG. 3. The first conductive member 40 and the second conductive member 50 are formed in a flanged bottomed cylindrical shape.

The first conductive member 40 includes a first bottomed cylindrical part 41 having an open end 41a at one end and a closed end 41b at the other end, and a first flange part 42 extending radially outward from the open end 41a of the first bottomed cylindrical part 41. The outer diameter of the first bottomed cylindrical part 41 is set to be substantially the same as the diameter of the through hole 10c of the support member 10. A fitting portion 43 recessed in the radial direction is formed in an area near the closed end 41b on the outer peripheral surface of the first bottomed cylindrical part 41. The first flange part 42 is slightly inclined so that the outer side in the radial direction is positioned closer to the closed end 41b of the first bottomed cylindrical part 41 than the inner side.

In the first conductive member 40, mainly a surface of the first flange part 42 opposite to the first bottomed cylindrical part 41 side is embedded in the electrode body 21 of the electrode member 20, the first bottomed cylindrical part 41 is inserted into the through hole 10c of the support member 10 (i.e., extends through the support member 10), and an area (including the fitting portion 43) of the first bottomed cylindrical part 41 on the closed end 41b side protrudes from the rear surface 10b of the support member 10. In the following, a surface of the first flange part 42 opposite to the first bottomed cylindrical part 41 is referred to as a "embedded surface".

The second conductive member 50 includes a second bottomed cylindrical part 51 having an open end 51a at one end and a closed end 51b at the other end, and a second flange part 52 extending radially outward from the open end 51a of the second bottomed cylindrical part 51. The second bottomed cylindrical part 51 is formed so as to increase the inner diameter toward the closed end 51b from the open end 51a. The inner diameter of the open end 51a of the second bottomed cylindrical part 51 is set to be substantially the same as the outer diameter of the fitting portion 43 formed on the outer peripheral surface of the first bottomed cylindrical part 41 of the first conductive member 40. The side surface and the bottom surface of the second bottomed cylindrical part 51 are connected by a smooth curved surface. The second flange part 52 has an inclined part that is inclined so that the outer side in the radial direction is away from the closed end 51b of the second bottomed cylindrical part 51 than the inner side.

In the second conductive member 50, the open end 51a of the second bottomed cylindrical part 51 is fitted and fixed to the fitting portion 43 of the first bottomed cylindrical part 41 of the first conductive member 40, by caulking or the like, thereby, the connector 30 of the biological electrode 1 is formed.

Next, an example of the manufacturing method of the biological electrode 1 will be described. However, the manufacturing method of the biological electrode 1 is not limited to the following method. In the following description, it is assumed that the connector 30 is attached to the support member 10 in advance. Specifically, it is assumed that the first bottomed cylindrical part 41 of the first conductive member 40 is inserted into the through hole 10c of the support member 10 from the closed end 41b side, and the open end 51a of the second bottomed cylindrical part 51 of the second conductive member 50 is fitted to the fitting portion 43 of the first bottomed cylindrical part 41 of the first conductive member 40 protruding from the rear surface 10b of the support member 10, thereby, the assembly of the support member 10 and the connector 30 is formed in advance. Therefore, in the following manufacturing method, the connector 30 is treated as a member provided on the support member 10 side.

In manufacturing the biological electrode 1, first, a conductive rubber which is in a liquid state or a paste state and contains silicone rubber and metal particles is stirred, and the stirred conductive rubber is injected into a mold (cavity) having a shape of the electrode member 20. In this way, the conductive rubber is formed in the shape of the electrode member 20 in the mold.

Next, the support member 10 to which the connector 30 is attached, that is, the assembly of the support member 10 and the connector 30 is placed on the conductive rubber in the mold with the support surface 10a of the support member 10 facing downward. Thereby, the support surface 10a of the support member 10 is placed on an area corresponding to the supported surface 21a of the electrode body 21 of the conductive rubber formed in the shape of the electrode member 20. In addition, the embedded surface of the first flange part 42 of the first conductive member 40 is embedded in an area corresponding to the electrode body 21 of the conductive rubber formed in the shape of the electrode member 20.

Next, the conductive rubber formed in the shape of the electrode member 20 is crosslinked, with the assembly of the support member 10 and the connector 30 placed thereon. Thus, the conductive rubber formed in the shape of the electrode member 20 is cured, and the first conductive member 40 and the electrode member 20 are integrated. That is, the connector 30 and the electrode member 20 having the electrode body 21 are integrally molded. In this way, the support member 10, the electrode member 20, and the connector 30 are integrated. Thereafter, the integrated support member 10, the electrode member 20, and the connector 30 are removed from the mold and post-processing as needed to manufacture the biological electrode 1.

### Industrial applicability

The biological electrode of the present invention can be used as a biological electrode for sensing electrical signals from a body of a subject, transmitting electrical stimulations to the subject, or both sensing and transmitting as described above, by contacting the body of the subject.

### Description of Reference Numerals

1: biological electrode
10: support member
10a: support surface
10b: rear surface
10c: through hole
20: electrode member
21: electrode body
21a: supported surface
21b: electrode protrusion forming surface
22: electrode protrusion
25: virtual circle
28: central part
29: outer circumferential part
30: connector
40: first conductive member
41: first bottomed cylindrical part
41a: open end
41b: closed end
42: first flange portion
43: mating part
50: second conductive member
51: second bottomed cylindrical part
51a: open end
51b: closed end
52: second flange portion
60: subject (for detection)
C1: center of the cross section of the proximal end of the electrode protrusion
C2: center of the distal end portion of the electrode protrusion
h: protrusion height
O: arrangement center of the plurality of electrode protrusions
Oz: measurement position
x: position

## Claims

1. A biological electrode (1) comprising an electrode member (20) in contact with a body of a subject (60), wherein
the electrode member (20) has an electrode body (21) having a plate shape and a plurality of electrode protrusions (22) provided so as to protrude from an electrode protrusion forming surface (21b) of the electrode body (21),
the plurality of electrode protrusions (22) are arranged on an outer peripheral part of the electrode protrusion forming surface (21b) so as to avoid a central part of the electrode protrusion forming surface (21b) of the electrode body (21), and
a protrusion height (h) of each electrode protrusion (22) from a proximal end thereof to a distal end thereof in a direction perpendicular to the electrode protrusion forming surface (21b) is 6 to 15mm, wherein
each of the plurality of electrode protrusions (22) has an oblique conical shape with a rounded apex,
each of the plurality of electrode protrusions (22) has a circular cross section which gradually decreases in diameter from the proximal end to the distal end, and
each of the plurality of electrode protrusions (22) is formed such that, when viewed from an arrangement center (O) of the plurality of electrode protrusions (22), a center (C2) of a cross section of the distal end is located radially outward of a center (C1) of a cross section of the proximal end,
**characterized in that**
each of the plurality of electrode protrusions (22) is formed such that, when viewed from the arrangement center (O) of the plurality of electrode protrusions (22), the center (C2) of the cross section of the distal end is located within the cross section of the proximal end.

2. The biological electrode (1) according to claim 1, wherein each of the plurality of electrode protrusions (22) is arranged so as to be located on the circumference of one virtual circle (25) or a plurality of virtual concentric circles on the outer peripheral part of the electrode protrusion forming surface (21b).

3. The biological electrode (1) according to any one of claims 1 and 2, wherein the plurality of electrode protrusions (22) are arranged at equal intervals on the outer peripheral part of the electrode protrusion forming surface (21b).

4. The biological electrode (1) according to any one of claims 1 to 3, which is suitable to be used for electroencephalography of a subject (60).

## Patentansprüche

1. Biologische Elektrode (1) mit einem Elektrodenbauteil (20), das mit einem Körper eines Subjekts (60) in Kontakt ist, wobei
das Elektrodenbauteil (20) einen Elektrodenkörper (21), der eine Plattenform hat, und eine Vielzahl von Elektrodenvorsprüngen (22) hat, die vorgesehen sind, um von einer elektrodenvorsprungsausbildenden Fläche (21b) des Elektrodenkörpers (21) vorzustehen,
die Vielzahl von Elektrodenvorsprüngen (22) an einem Außenumfangsteil der elektrodenvorsprungsausbildenden Fläche (21b) angeordnet sind, um einen zentralen Teil der elektrodenvorsprungsausbildenden Fläche (21b) des Elektrodenkörpers (21) zu vermeiden, und
eine Vorsprungshöhe (h) von jedem Elektrodenvorsprung (22) von einem proximalen Ende von diesem zu einem distalen Ende von diesem in einer Richtung senkrecht zu der elektrodenvorsprungsausbildenden Fläche (21b) 6 bis 15 mm ist, wobei
jeder der Vielzahl von Elektrodenvorsprüngen (22) eine schräge konische Form mit einer abgerundeten Spitze hat,
jeder der Vielzahl von Elektrodenvorsprüngen (22) einen kreisförmigen Querschnitt hat, dessen Durchmesser sich von dem proximalen Ende zu dem distalen Ende allmählich verringert, und
jeder der Vielzahl von Elektrodenvorsprüngen (22) derart ausgebildet ist, dass, aus Sicht von einem Anordnungszentrum (O) der Vielzahl von Elektrodenvorsprüngen (22), ein Zentrum (C2) eines Querschnitts des distalen Endes radial außen von einem Zentrum (C1) eines Querschnitts des proximalen Endes gelegen ist,
**dadurch gekennzeichnet, dass**
jeder der Vielzahl von Elektrodenvorsprüngen (22) derart ausgebildet ist, dass, aus Sicht von dem Anordnungszentrum (O) der Vielzahl von Elektrodenvorsprüngen (22), das Zentrum (C2) des Querschnitts des distalen Endes innerhalb des Querschnitts des proximalen Endes gelegen ist.

2. Biologische Elektrode (1) gemäß Anspruch 1, wobei jeder der Vielzahl von Elektrodenvorsprüngen (22) angeordnet ist, um auf dem Umfang von einem virtuellen Kreis (25) oder einer Vielzahl von virtuellen konzentrischen Kreisen an dem Außenumfangsteil der elektrodenvorsprungsausbildenden Fläche (21b) gelegen zu sein.

3. Biologische Elektrode (1) gemäß einem der Ansprüche 1 und 2, wobei die Vielzahl von Elektrodenvorsprüngen (22) in gleichen Abständen an dem Außenumfangsteil der elektrodenvorsprungsausbildenden Fläche (21b) angeordnet sind.

4. Biologische Elektrode (1) gemäß einem der Ansprüche 1 bis 3, die geeignet ist, um für eine Elektroenzephalographie eines Subjekts (60) verwendet zu werden.

## Revendications

1. Électrode biologique (1) comprenant un élément d'électrode (20) en contact avec un corps d'un sujet (60), dans laquelle
l'élément d'électrode (20) comporte un corps d'électrode (21) ayant une forme de plaque et une pluralité de saillies d'électrode (22) prévues de manière à faire saillie à partir d'une surface de formation de saillies d'électrode (21b) du corps d'électrode (21),
la pluralité de saillies d'électrode (22) sont disposées sur une partie périphérique extérieure de la surface de formation de saillies d'électrode (21b) de manière à éviter une partie centrale de la surface de formation de saillies d'électrode (21b) du corps d'électrode (21), et
une hauteur de saillie (h) de chaque saillie d'électrode (22) depuis son extrémité proximale jusqu'à son extrémité distale dans une direction perpendiculaire à la surface de formation de saillies d'électrode (21b) est comprise entre 6 et 15 mm, dans lequel
chacune de la pluralité de saillies d'électrode (22) a une forme conique oblique avec un sommet arrondi,
chacune de la pluralité de saillies d'électrode (22) a une section transversale circulaire dont le diamètre diminue progressivement de l'extrémité proximale à l'extrémité distale, et
chacune de la pluralité de saillies d'électrode (22) est formée de telle sorte que, lorsqu'on la regarde depuis un centre d'agencement (O) de la pluralité de saillies d'électrode (22), un centre (C2) d'une section transversale de l'extrémité distale est situé radialement à l'extérieur d'un centre (C1) d'une section transversale de l'extrémité proximale,
**caractérisé en ce que**
chacune de la pluralité de saillies d'électrode (22) est formée de telle sorte que, vue depuis le centre d'agencement (O) de la pluralité de saillies d'électrode (22), le centre (C2) de la section transversale de l'extrémité distale est situé à l'intérieur de la section transversale de l'extrémité proximale.

2. Électrode biologique (1) selon la revendication 1, dans laquelle chacune de la pluralité de saillies d'électrode (22) est disposée de manière à être située sur la circonférence d'un cercle virtuel (25) ou d'une pluralité de cercles concentriques virtuels sur la partie périphérique extérieure de la surface de formation de saillies d'électrode (21b).

3. Électrode biologique (1) selon l'une quelconque des revendications 1 et 2, dans laquelle la pluralité de saillies d'électrode (22) sont disposées à intervalles égaux sur la partie périphérique externe de la surface de formation de saillies d'électrode (21b).

4. Électrode biologique (1) selon l'une quelconque des revendications 1 à 3, qui est adaptée pour être utilisée pour l'électroencéphalographie d'un sujet (60).
